# EUROPEAN PATENT SPECIFICATION

(11) **EP 0 794 807 B1**
(45) Date of publication and mention of the grant of the patent: **07.05.2003**
(21) Application number: 96932711.3
(22) Date of filing: 03.10.1996
(51) Int. Cl.: A61M 16/00

(54) **ARTIFICIAL AIRWAY DEVICE**
KÜNSTLICHE LUFTWEGVORRICHTUNG
DISPOSITIF D'INTUBATION POUR LA RESPIRATION ARTIFICIELLE

(30) Priority: 03.10.1995 GB 9520141; 01.03.1996 US 609521
(43) Date of publication of application: 17.09.1997
(73) Proprietor: Brain, Archibald Ian Jeremy, Chertsey, Surrey KT16 0DJ (GB)
(72) Inventor: Brain, Archibald Ian Jeremy, Chertsey, Surrey KT16 0DJ (GB)
(74) Representative: West, Alan Harry
(86) International application number: GB9602425
(87) International publication number: WO97012640

(56) References cited:
- WO-A-94/02191
- FR-A- 2 293 222
- GB-A- 1 399 093
- US-A- 5 241 956
- US-A- 5 305 743
- US-A- 5 355 879

## Description

This invention relates to a laryngeal-mask airway (LMA) device, which is an artificial airway device designed to facilitate lung ventilation in an unconscious patient by forming a low-pressure seal around the laryngeal inlet. An inflatable-ring seal surrounds an appropriately shaped mask which fits into the lower pharynx and is attached to a tube which emerges from the mouth, as for connection to medical gas-supply tubing.

More particularly, the invention relates to a variety of laryngeal masks, known as gastro-laryngeal masks (GLM), wherein provision is made for airway assurance to the patient who is at risk from vomiting or regurgitation of stomach contents while unconscious. U.S. Patent 5,241,956 deals with this problem by providing an evacuation tube which is open through the center of the inflatable seal of the laryngeal mask, thus utilizing the distal end of the inflatable ring as an inflatable-cuff formation which establishes peripherally sealed engagement to the upper sphinctral region of the oesophagus and centrally supports the distal end of the evacuation tube. In addition, said U.S. Patent 5,241,956 discloses a further inflatable cuff carried by the laryngeal mask and by the evacuation tube, for referencing inflation against the back wall of the pharynx, thus making it possible to establish the laryngeal-inlet seal with reduced inflation pressure, as compared with prior structures not having such an additional inflatable cuff.

U.S. Patent 5,305,743 discloses moulding techniques for manufacture of a variety of laryngeal masks, including a gastro-laryngeal mask, wherein an inflatable back cushion provides such referencing inflation against the back wall of the pharynx as to widely distribute the back-wall reference, over substantially the full area of the laryngeal mask. Such a back-cushion construction has been found to be mechanically simple and highly effective, and U.S. Patent 5,355,879 discloses such a back cushion for each of several representative laryngeal-mask constructions.

In practice, although a gastro-laryngeal-mask as described in said Patent 5,355,879 works well, it has the disadvantage that the gastric evacuation channel needs to be sufficiently stiff to prevent its collapse under the influence of the increased pressure within the back-cushion cuff, when it is inflated in the pharynx. A suitably stiff tube is readily provided, but the whole device is then more difficult to insert into the patient's throat, since insertion involves flexing the device around the angle at the back of the tongue. Provision of a pre-curved airway tube facilitates passage around the back of the tongue, but the advancing distal tip end of the device is then more likely to collide with the glottis (or entrance to the larynx), and indeed it may block the larynx by so doing, with consequent danger to the patient.

The present invention seeks to provide a laryngeal mask construction that specifically avoids the problems and difficulties encountered with the devices of the above U.S. patents and especially of U.S. Patent 5,355,879 by allowing for ready compression and flexure of a gastric passage within a back-cushioned or cuffed gastro-laryngeal mask when the mask is in deflated condition for insertion into the patient's throat.

Such a construction has been found to enable formation of a flattened flexible leading distal-end edge to self-adapt to and resiliently ride the outer limit of curvature of the patient's airway, throughout the insertional course of the deflated mask and into its locating engagement with the hypopharynx, and to provide for assurance of full patency of the gastric passage within the mask, when the mask has been inflated.

These objectives are realized by utilizing two structural mechanisms, both of which are operative when the device is inflated; one of these mechanisms prevents lateral compression of the wall of the gastric tube, while the other prevents antero-posterior compression of the wall of the gastric tube. The result is to assure a substantially circular section within relatively soft portions of the evacuation passage as long as the device is inflated and in installed position.

According to the invention, there is now provided a laryngeal mask airway device comprising
a generally elliptical inflatable ring of relatively thin and softly pliant molded elastomeric material, having an outer periphery adapted for sealed engagement to the laryngeal inlet and including within its inner periphery an apertured panel separating its pharyngeal and laryngeal sides, the ring extending longitudinally between proximal and distal ends and having an inflation port connection at its proximal end, the ring further including at its distal end an open tubular conduit for operative engagement and communication with the oesophageal inlet, the tubular conduit extending from its distally open end proximally on the pharyngeal side of the panel;
a domed backing-plate of relatively stiff material having a concave side terminating in a generally elliptical footing in a geometric plane and sealed around the aperture in the panel at the inner periphery of the elliptical ring and having a proximal airway-tube connector at an acute angle with the panel, the backing plate providing stability to the inner periphery of the elliptical ring and directional stability for the tubular conduit;
an airway tube connected to the connector,
a gastric-discharge tube connected to the tubular conduit; and
an inflatable back cushion comprising a panel of softly compliant material bonded peripherally to the pharyngeal side of the ring and extending over the tubular conduit;
characterized in that
the posterior aspect of the tubular conduit is bonded along its length to the back cushion panel;
the anterior aspect of the tubular conduit is bonded along its length to the backing plate; and
lateral aspects of the tubular conduit are reinforced with circumferentially arcuate ribs.

In a preferred embodiment of the invention, an artificial airway device to facilitate a patient's lung ventilation comprises an airway tube, an evacuation tube, and a laryngeal mask at one end of both tubes. The mask is of generally elliptical configuration and comprises a body or backplate of relatively stiffly compliant nature, and an inflatable annular cuff or ring of relatively softly compliant nature is connected to and surrounds the body or backplate. When inflated, the annular cuff adapts to and seals around the laryngeal inlet, and an inflatable cushion on the exterior of the inflated annulus bears against the back wall of the pharynx, to thereby forwardly load the inflated annulus into sealed relation with the laryngeal inlet, with the backplate dividing the mask between a laryngeal-chamber side and a pharyngeal-chamber side. The relatively stiff backplate is formed for connection to the airway tube for exclusive communication to the larynx through an opening in the backplate; and the backplate is also, configured to guide and support a relatively soft flexible evacuation tube within the pharyngeal-chamber side, from a distally open end for reception of gastric products, to a proximal end for connection to an externally discharging evacuation tube.

It is a feature of the invention that along an aligning path for the flexible evacuation tube within the pharyngeal-chamber side of the mask, a first significant angular fraction of the periphery of the flexible tube is bonded to a stabilizing portion of the backplate, and that a second angular fraction of the periphery of the flexible tube is continuously bonded to the inner surface of the flexible back cushion, such that generally opposite unbonded further angular regions exist between the bonded regions. These unbonded further regions are provided with external stiffening ribs at a succession of axial intervals, to reinforce the unbonded regions against lateral compression when the back cushion and the inflatable ring are under inflation pressure. In this way, inflation of the annular laryngeal-inlet sealing ring and of the flexible back cushion will assure a maximally open evacuation passage within the mask in inflated condition, essentially without antero-posterior or lateral compression of the passage. And it is further assured that upon deflation of the mask, evacuation-passage compression will be essentially in the sense of achieving a squeezing and somewhat flattening deformation of the discharge passage against the formed back-plate area of evacuation-passage support; such flattening is maximal at the oesophageal end of the discharge passage, so that, when correctly deflated, the device forms a wedge shape for correct insertion.

The invention will be illustratively described in detail for a presently preferred embodiment, and for certain other embodiments, all in conjunction with the accompanying drawings, in which:
Fig. 1 is a simplified view, generally in side elevation, for the presently preferred embodiment of an artificial airway device, having at its distal end a laryngeal mask with a gastric-drainage feature of the invention, the same being shown in position for use in a patient;
Fig. 2 is a fragmentary plan view, to an enlarged scale showing the back or pharynx-facing side of the mask of Fig. 1;
Fig. 3 is a plan view to the scale of Fig. 2, showing a softly compliant moulded inflatable component of the mask, as seen from the aspect of Fig. 2;
Fig. 4 is a plan view to the scale of Fig. 2, showing a relatively stiffly compliant rigidising or reinforcing back-plate component of the mask, as seen from the aspect of Fig. 2;
Fig. 5 is a longitudinal section of the softly compliant component of Fig. 3, to the scale of Figs. 2 to 4 and taken generally in the vertical plane 5-5 of substantial symmetry, but prior to an inside-out deformation step, to create the appearance of Fig. 3;
Fig. 6 is another view in longitudinal section, to the scale of Figs. 2 to 5 and in the vertical plane 5-5 of Fig. 3, showing the relatively stiff component of Fig. 4 in assembled relation to the softly compliant component of Fig. 3;
Fig. 7 is a side perspective view, of the proximal end of the rigidising component of Fig. 4;
Fig. 8 is a simplified cross-sectional view of the inflated mask of Fig. 2, taken at 8-8 in Fig. 2;
Fig. 9 is a simplified cross-sectional view of the deflated mask of Fig. 2, taken at 8-8 in Fig. 2;
Fig. 10 is a view similar to Fig. 2, to show a first modification;
Fig. 11 is a view similar to Fig. 4, to show the back-plate component in the modification of Fig. 10; and
Fig. 12 is a sectional view, taken at 12-12 in Fig. 11.

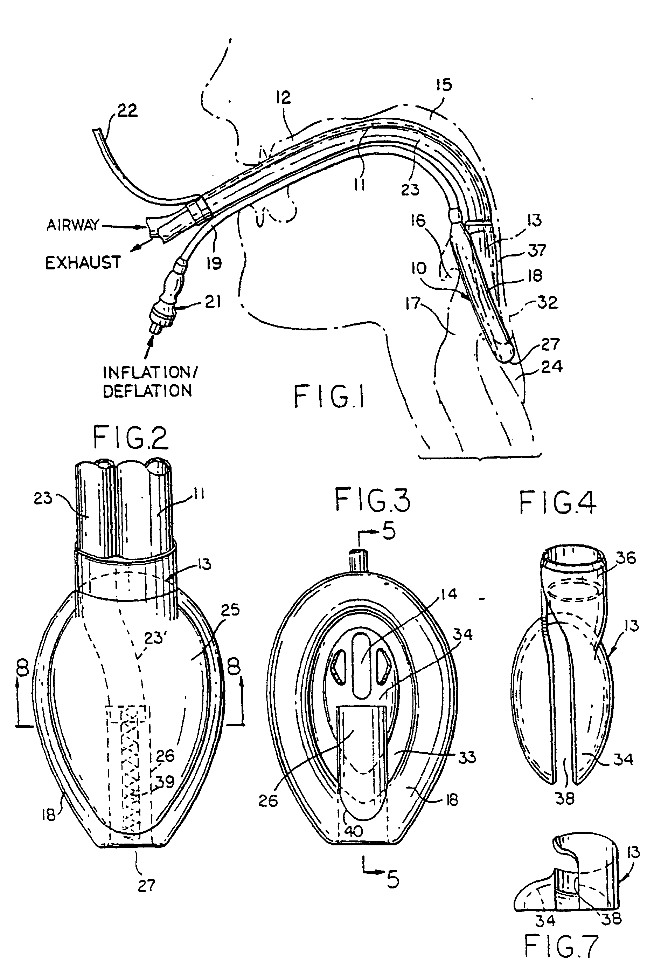

Referring first to the preferred embodiment of Figs. 1 to 9, the invention is shown in application to an airway system comprising a laryngeal-mask unit 10 and its airway tube 11, installed through the mouth 12 of a patient. The mask unit 10 may be generally as described in any of the above-identified U.S. patents and therefore need not now be described in detail. It suffices to say that mask unit 10 comprises a body portion 13 having a lumen 14 through which the airway tube 11 can establish a free externally accessible ventilation passage, via the patient's mouth 12 and throat 15, and past the epiglottis 16 to the larynx 17. The body 13 of mask 10 may be of an elastomer such as silicone rubber and relatively stiff; and body 13 is surrounded by an inflatable ring 18 which is generally elliptical and which is circumferentially united to body 13 in essentially a single plane. The inflatable ring 18 may also be of silicone rubber, although preferably relatively soft and flexible compared to body 13. An externally accessible tube 19 is the means of supplying air to the inflatable ring 18 and of extracting air from (and therefore collapsing) ring 18 for purposes of insertion in or removal from the patient; check-valve means 21 in tube 19 will be understood to hold a given inflation or to hold a given deflation of ring 18.

In the installed position of Fig. 1, the projecting but blunted distal end 27 of ring 18 is shaped to conform with the base of the hypopharynx where it has established limited entry into the upper sphinctral region of the oesophagus 24. The back side of body 13 is covered by a thin flexible panel 25 (Fig. 2) which is peripherally bonded to the inflatable ring 18 (Fig. 1) to define an inflatable back cushion which assures referencing to the back wall of the pharynx and thus is able to load the mask unit forward for enhanced effectiveness of inflated-ring sealing engagement to the laryngeal inlet. The inflated ring, thus-engaged to the laryngeal inlet, orients the distal-end of the airway tube 11 at an acute angle to the general plane of ring 18 and in substantial alignment with the axis of the laryngeal inlet, for direct airway communication only with the larynx 17.

The laryngeal-mask unit 10 is of the GLM variety in which an evacuation tube 23 (Fig. 2) serves for extraction and external removal of gastric-discharge products from the oesophagus. Tube 23 follows the general course of the airway tube 11, with sealed entry alongside airway tube 11, beneath the back-cushion panel 25, and with passage through the interior of ring 18, near the distal end of the mask; in Fig. 3, the distally open end of the evacuation tube 23 is defined by a re-entrant tubular formation 26 integrally formed with the relatively soft material of ring 18. As explained in U.S. Patent 5,241,956, inflation-air supply to the back cushion may be the same (19) as for ring 18, or separate inflating means (not shown) may be provided for these separate inflatable means.

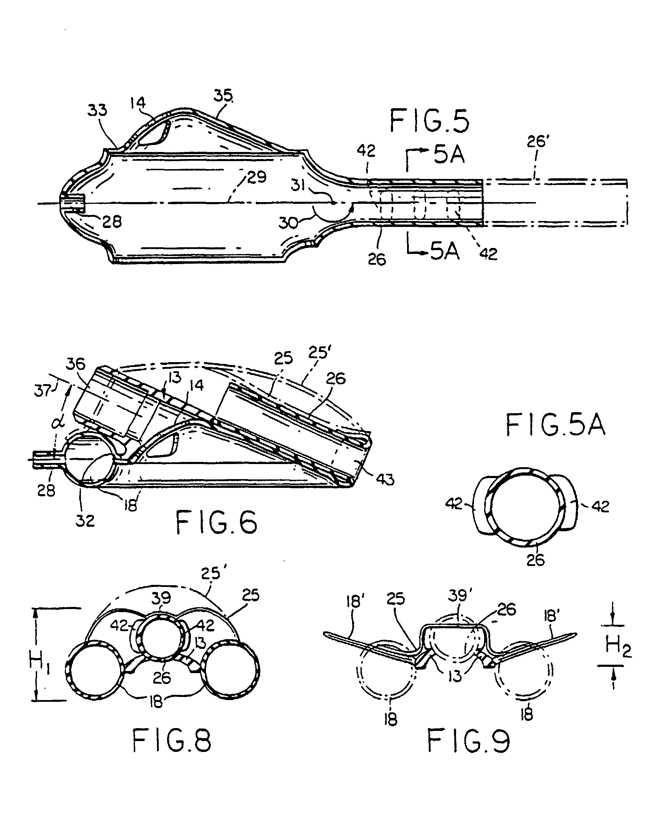

More specifically, for the particular construction shown, the relatively softly compliant flexible components may be integrally formed in a single moulding operation, in which the moulded intermediate product is an inside-out version of what will become the finished more flexible part of the finished mask unit 10. The moulded intermediate product may thus have the appearance shown in Fig. 5, following the technique described in U.S. Patent 5,305,743, to which reference is made for detailed description. It suffices here to identify the inflation-air inlet formation 28, directed inwardly on a central axis 29 which also includes the outwardly directed distal-end formation of the evacuation tube 26; the central axis 29 may also be understood as identifying the equator plane (perpendicular to the drawing of Fig. 5) which applies to the inflatable annular ring 18, after evacuation tube 26 has been swung upward (counterclockwise), in the sense suggested by arrow 30, and generally for 180° of rotation about an axis 31, which (axis 31) is normal to the plane of the drawing of Fig. 5. This 180° rotation tucks tube 26 into the flanged relatively large edge 32 of the open skirt of the moulded intermediate product of Fig. 5 and makes it a simple matter to turn the remainder of the skirt inside-out, thus defining ring 18, with the edge flange 32 seated on a ledge 33 of the upper dome-shaped feature of the moulded intermediate product.

In the preferred form shown, the mask body 13 (Figs. 4 and 7) is a separately moulded component of relatively stiff nature as compared to the moulded intermediate product of Fig. 5. Stiffness *vs*. softness will be understood to be relative terms and not necessarily to imply that these components are formed from different materials.

The body component 13 comprises essentially a moulded dome or bowl 34 having a concave inner surface which conforms to the convex moulded contour of the dome shape 35 of the relatively soft (*i.e*., thin-walled) component of Fig. 5, these components being shown in Fig. 6 in assembled relation. Relative stiffness (thickness) in the bowl or dome 34 of Fig. 4 is generally in the range 2 to 5 mm, with gradually reducing thickness for greater flexibility in approach to the lower or distal end. The bowl or dome 34 has a peripheral edge which terminates in a single plane, for adhesively bonded seating to the ledge 33 of the relatively soft component of Fig. 5, after making the inside-out inversion.

The stiffness of body 13 is greatest in the region of proximal-end seating to ledge 33, above which an inlet-air formation 36 is oriented on an axis 37 which is not only inclined at an acute angle α to the plane of seating to ledge 33, but is also laterally offset from the central longitudinal plane of symmetry of the mask, denoted 5-5 in Fig. 3. Relative stiffness of body 13 is also enhanced (i) by the fact that its distal half features a slot 38 of width less than the diameter of the re-entrant distal-end tube 26, (ii) by the fact that the re-entrant tube 26 is adhesively retained in cradled support by and between confronting edges of slot 38, and (iii) by the fact that the distal end of evacuation tube 23 is preferably preformed (as seen in Fig. 2) with a quarter-turn helical advance to track the course of slot 38 in the upper or proximal half of body 13. The evacuation tube 23 is preferably relatively stiff, *e.g*., stiffness (thickness) in the order of magnitude of the material at the upper (proximal) half of body 13, and is seen in Fig. 2 to have telescoping fit to the proximally directed upper end of re-entrant tube 26; this is an adhesively sealed fit.

As also seen in Fig. 2, the back-cushion panel 25 covers a substantial part of the posterior surface of the mask, being peripherally sealed around the generally elliptical course of inflatable ring 18, and also being centrally adhered to the re-entrant tube 26 for substantially the entire length of tube 26, as suggested by cross-hatching 39. Finally, to assure integrity of the inflatable ring 18, the re-entrant tube 26 is adhesively sealed to the adjacent edges of tube-26 local passage through ring 18 at the distal location designated 40 in Fig. 3; for purposes of avoiding undue complexity in the drawings, this adhesively sealed region is not shown but will be understood to be along the line of tube-26 intercept with locally adjacent walls of inflatable ring 18. In Fig. 5, this intercept line is accounted for by a local cut-out 40' at the distal end of the skirt of the intermediate product of Fig. 5.

The simplified sectional diagram of Fig. 8 illustrates the functional cooperation of described component parts and features of the described gastro-laryngeal mask construction, in inflated condition, to account for diametrically opposite section cuts through right and left halves of the inflatable ring 18, spaced by body-13 sealed fit to the inner profile of ring 10. The back-cushion panel 25, being centrally adhered at 39 to the upper central region of re-entrant tube 26, provides a lifting force which is in the direction to hold open the evacuation tube and, therefore, not to collapse tube 26 when the back cushion is inflated; without this force, in opposition to a retaining force attributable to adhesive connection to body 13 (along edges of slot 38), there would be no tendency to hold a softly compliant tube 26 against collapse, in that the cushion panel would outwardly expand itself to a bowed shape 25' suggested by phantom outline in Fig. 2.

Preferably, the effective arcuate extent of_adhesive connection 39 is in the range 45° to 90° about the central axis of tube 26, as seen in Fig. 8. Preferably also, the adhesive connection of tube 26 along the straight edges of the distal half of slot 38 accounts for a corresponding range of support of tube 26 against collapse in the circumstance of back-cushion inflation. In other words, inflation of the ring 18 and back cushion 25 will assure developed vertical forces to hold the evacuation passage of re-entrant tube 26 in substantially open condition, but the transversely opposed arcuate regions (each of approximately 90° arcuate extent) between these adhesively connected regions are vulnerable to compressionally inward bowing, thus reducing the sectional area of tube 26 while the mask is inflated. The invention resolves this vulnerability by providing axially spaced stiffening ribs or ridges 42 as integral formations of the re-entrant tube 26, in the initially moulded intermediate product of Fig. 5. As shown, there are three mutually opposed pairs of ridges 42, at axial spacings which are in the order of the unstressed bore diameter of tube 26. For the indicated silicone-rubber material of the product of Fig. 5, the incremental local thickness at ridges 42 is suitably twice or three times the otherwise uniformly thin moulded product of Fig. 5, as seen in Fig. 5A.

In Fig. 8, a section taken near the location of tube 26 connection to the more stiffly compliant evacuation tube 23, the inflated condition of the GLM mask of the invention is seen to have an overall "height" dimension H₁, meaning front-to-back (*i.e*., laryngeal inlet-to-pharynx back wall). When the mask is deflated, this dimension H₁ is seen to be reduced by approximately 50 percent, as shown at H₂ in Fig. 9 for the deflated condition of the same mask. When deflated, as has been pointed out in U.S. Patent 5,297,547, the ring 18 collapses into-flattened double walls (marked 18') which are upwardly dished; and although deflation does little to compress tube 26 other than at the region 39' of adhesion to the back-cushion panel 25, the overall deflated extent H₂ is essentially unchanged from the dimension H₂ which applies for collapse of ring 18. On the other hand, at the distal end of the mask, the collapse of ring 18 is operative upon the formed distal-end opening 43 of tube 26 to somewhat flatten the opening 43, into a generally shovel-shaped distal lip feature which merges smoothly into the adjacent upwardly dished double-wall shape 18' shown in the longitudinal mid-section of Fig. 9.

It will be appreciated that the GLM device described thus far has an airway tube 11 that is of larger diameter than the evacuation tube 23; in this circumstance, the airway tube 11 is large enough to accommodate guided insertion of an endotracheal tube. The tubes 11, 23 enter the described laryngeal mask 10 in side-by-side relation and are preferably adhesively secured to each other in this side-by-side relation, and along their full longitudinal extent, in order to provide a measure of torsional resistance against twisting, thereby aiding a medically qualified person in quickly and correctly installing a fully deflated GLM in a patient, with assurance that, upon inflation of ring 18 and the back-cushion panel 25, an exclusive and sealed airway connection will be established to the laryngeal inlet, *via* lumen 14 and from the airway tube 11; concurrently, a similarly exclusive evacuation connection is established to the upper sphinctral region of the oesophagus, *via* the distal-end opening 43 of tube 26, through the evacuation tube 23, and to suitable waste-collection means (not shown) external to the patient.

More specifically as to insertion of the fully deflated GLM device in a patient, it will be understood that a range of GLM sizes is available from which to select a sufficiently correct size for the patient. Deflation is accomplished *via* external means (not shown) and *via* check-valve means 21 to hold the deflated condition wherein the dome shape of body 13 rises from within the dished peripheral lip 18' of the collapsed ring 18. A skilled operator is quickly able to develop the desired appearance of the GLM in its deflated state; but for a uniformly correct deflated shaping, it is recommended to use a forming tool.

When correctly shaped and in its deflated condition, and at the distal end of the GLM, the opening 43 will have been flattened, and this distal end merges with the peripheral lip 18' of the collapsed ring 18. Noting that the entire distal half of the mask is of relatively soft material, stiffened only by indicated adhesive connection, the distal end projects distally and at its upwardly flared merge with lip 18', for low acute-angle incidence to the posterior arcuate profile of the patient's throat passage. That being the case, a medical technician need only make sure that upon inserting the mask *via* the patient's mouth and throat, the flattened distal end rides the outer (posterior) arcuate contour of the patient's airway, in that the softly flexible nature of the distally projecting and somewhat flattened distal end will be flexibly self-adapting to local irregularities (if any) in the course of passage into the pharynx; final insertional location is noted by an increase in encountered resistance, upon distal-end engagement of the GLM with the upper sphinctral region of the oesophagus. At this juncture, inflation air supplied *via* line 19 and retained by check-valve means 21 establishes (i) the described seal of ring 18 to the laryngeal inlet, (ii) back cushion (panel 25) contact with the back wall of the pharynx, and (iii) full opening of the evacuation tube 26 for maximum accommodation of a possible gastric discharge from the oesophagus.

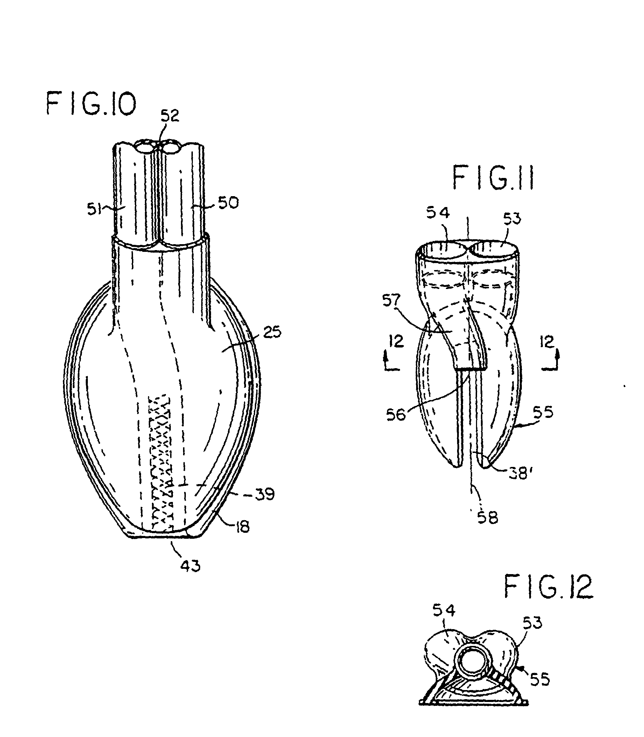

Beyond what has been described, Fig. 10 illustrates at phantom outline 26' that the flexible length of the re-entrant tube 26 may be of even greater length than the approximately half-mask length shown by the solid lines of Fig. 5. In that event, arcuate stiffener ridges as described at 42 will be preferred, as long as lateral support is needed to prevent side-wall collapse of the extended tube 26', in the inflated condition of the mask, *i.e.*, including inflation of back-cushion panel 25.

Figs. 10 to 12 illustrate another GLM embodiment wherein an airway tube 50 and an evacuation tube 51 are of equal size, adhered (as suggested at 52) to each other in side-by-side relation for torsionally resistant and symmetrically positioned entry into corresponding side-by-side ports 53, 54 of the moulded backing plate 55 of Figs. 11 and 12. The backing plate 55 may be similar to plate 13 of Fig. 4, except that in Fig. 11 the somewhat helically arcuate conduit path from the inserted distal end of evacuation tube 51 to the point 56 of softly compliant re-entrant tube (26) connection is provided by an integral passage formation 57 of the backing plate 55. At point 56 in Fig. 11, the formation 57 is seen to be in the central vertical plane 58 of symmetry of the bowl of backing plate 55 and in alignment for accepted proximal-end insertional accommodation of a re-entrant tube 26 of thin-walled material to which backing plate 55 is to be assembled, with edges of the straight slot 38' supporting tube 26 in the manner already described. Also integrally formed with backing plate 55 is an inlet-connection counterbore for coupled connection of airway tube 50 to the laryngeally exposed side of the mask. Features in Fig. 10, such as the back-cushion panel 25, the inflatable ring 18, and the adhesively bonded connection 39 of panel 25 to tube 26 are all as previously described.

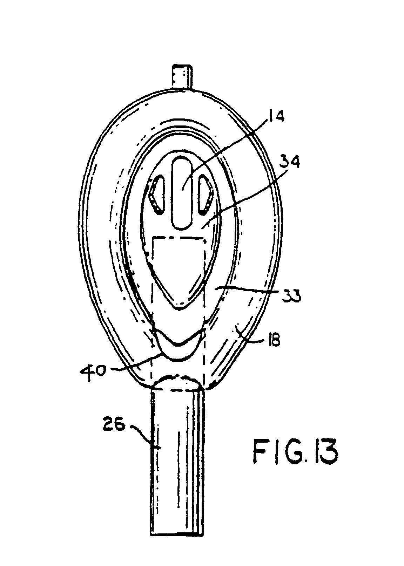

It will be understood that the inside-out technique described in connection with Figs. 5 and 6 for initially moulding and then inverting the skirt of the moulded product, is but one illustration of a way to create the mask and its inflatable ring. In which case the flexible drainage conduit does not get inverted. That being the case, the reinforcement ribs 42 are initially formed portions of the outer surface of the moulded product. On the other hand, another technique for forming the mask with its inflatable ring, involves moulding the mask bowl integrally with an elliptically configured product as shown in Fig. 13, wherein completion of inflatable-ring (18) integrity requires only an adhesively bonded completion of the ring peripherally around the inner substantially elliptical profile, where backing-plate (13) connection is also adhesively secured. In that case, the drainage tube 26 is integrally-moulded with the non-invertible ring (18), so that an inversion of tube 26 is necessary, to have it project re-entrantly, in the proximal direction, and the moulded product which is to become inflatable ring 18 must be cut away as at 40, to permit inverted tube 26 to "pass through" the inflatable ring, in order to develop a relationship which is suggested by Fig. 5. Of course, if tube 26 is to be inverted, the reinforcement ribs 42 are preferably integrally formed as radially inward rib reinforcements or discontinuities in the moulded bore of tube 26. Inversion of tube 26 places these rib reinforcements on the outer surface of tube 26, so that the bore of tube 26 is inherently smooth.

## Claims

1. A laryngeal mask airway device comprising:
a generally elliptical inflatable ring (18) of relatively thin and softly pliant molded elastomeric material, having an outer periphery adapted for sealed engagement to the laryngeal inlet and including within its inner periphery an apertured panel (33) separating its pharyngeal and laryngeal sides, the ring extending longitudinally between proximal and distal ends and having an inflation port connection (28) at its proximal end, the ring further including at its distal end an open tubular conduit (26) for operative engagement and communication with the oesophageal inlet, the tubular conduit extending from its distally open end proximally on the pharyngeal side of the panel;
a domed backing-plate (34) of relatively stiff material having a concave side terminating in a generally elliptical footing in a geometric plane and sealed around the aperture in the panel (33) at the inner periphery of the elliptical ring and having a proximal airway-tube connector (36) at an acute angle with the panel, the backing plate providing stability to the inner periphery of the elliptical ring and directional stability for the tubular conduit;
an airway tube (11) connected to the connector (36);
a gastric-discharge tube (23) connected to the tubular conduit (26); and
an inflatable back cushion (25) comprising a panel of softly compliant material bonded peripherally to the pharyngeal side of the ring (18) and extending over the tubular conduit (26);
**characterized in that**
the posterior aspect of the tubular conduit is bonded along its length to the back cushion panel;
the anterior aspect of the tubular conduit is bonded (39) along its length to the backing plate; and
lateral aspects of the tubular conduit are reinforced with circumferentially arcuate ribs (42).

2. A device according to claim 1, wherein the airway tube (11) and the gastric-discharge tube (23) are bonded together side-by-side.

3. A device according to claim 1 or claim 2, wherein the tubular conduit (26) extends proximally up to 50 percent of the longitudinal extent of the inflatable ring.

4. A device according to claim 1 or claim 2, wherein the tubular conduit (26) extends proximally more than 50 percent of the longitudinal extent of the inflatable ring.

5. A device according to any one of claims 1 to 4, wherein the backing-plate (34) guides the tubular conduit (26) in a straight proximal direction for substantially the distal half of the longitudinal extent of the mask.

6. A device according to claim 5, wherein the backing-plate member (34) guides the tubular-conduit (26) on a generally helical arc to a proximal location connecting with the gastric-discharge tube (23) alongside the airway tube (11).

7. A device according to any one of claims 1 to 6, wherein the back cushion panel is tangentially bonded (39) to the tubular conduit.

8. A device according to claim 7, wherein the back cushion bond (39) to the tubular conduit extends for substantially the distal half of the longitudinal extent of the inflatable ring.

9. A device according to any one of claims 1 to 8, wherein the arcuate ribs (42) are axially spaced.

10. A device according to claim 9, wherein the ribs (42) project radially outwardly of the tubular conduit.

## Patentansprüche

1. Kehlkopfmasken-Luftwegvorrichtung mit
einem generell elliptischen, aufweitbaren Ring (18) aus relativ dünnem und weich-biegsam geformtem Elastomermaterial, der einen zu dichtendem Eingriff mit dem Kehlkopfeingang ausgelegten Außenumfang aufweist und innerhalb seines Innenumfangs eine Platte (33) mit Öffnung aufweist, die seine Schlund- und Kehlkopfseite voneinander trennt, wobei der Ring longitudinal zwischen proximalem und distalem Ende verläuft und ein Aufweitungsportanschlußstück (28) an seinem proximalen Ende aufweist und ferner an seinem distalen Ende eine offene Rohrleitung (26) zum operativen Eingriff und kommunizierend mit dem Speiseröhreneingang, wobei die Rohrleitung von ihrem distalen offenen Ende aus proximal auf der Schlundseite der Platte verläuft;
einer gewölbten Rückwand (34) aus relativ steifem Material, die eine konkave Seite aufweist, die in einer generell elliptischen Fußfläche in einer geometrischen Ebene endet und rund um die Öffnung der Platte (33) am Innenumfang des elliptischen Rings abgedichtet ist, und die ein proximales Luftwegrohr-Anschlußstück (36) in einem spitzen Winkel zu der Platte aufweist, wobei die Rückwand dem Innenumfang des elliptischen Rings Stabilität und der Rohrleitung Richtungsstabilität verleiht;
einem an das Verbindungsstück (36) angeschlossenen Luftwegrohr (11) ;
einem an die Rohrleitung (26) angeschlossenen Magenentleerungsrohr (23); und
einem aufweitbaren Rückkissen (25), das eine Platte aus weich-nachgiebigem Material aufweist, die umfangsmäßig an der Schlundseite des Rings (18) befestigt ist und über die Rohrleitung (26) verläuft;
**dadurch gekennzeichnet, daß**
das Hinterende der Rohrleitung längsseitig an der Rückkissenplatte befestigt ist;
das Vorderende der Rohrleitung längsseitig an der Rückwand befestigt (39) ist; und
die Seitenenden der Rohrleitung mit umlaufenden bogenförmigen Rippen (42) verstärkt sind.

2. Vorrichtung nach Anspruch 1, wobei das Luftwegrohr (11) und das Magenentleerungsrohr (23) Seite an Seite aneinander befestigt sind.

3. Vorrichtung nach Anspruch 1 oder 2, wobei die Rohrleitung (26) proximal über bis zu 50 % der longitudinalen Erstreckung des aufweitbaren Rings verläuft.

4. Vorrichtung nach Anspruch 1 oder 2, wobei die Rohrleitung (26) proximal über mehr als 50 % der longitudinalen Erstreckung des aufweitbaren Rings verläuft.

5. Vorrichtung nach einem der Ansprüche 1 bis 4, wobei die Rückwand (34) die Rohrleitung (26) für im wesentlichen die distale Hälfte der longitudinalen Erstreckung der Maske in eine gerade proximale Richtung führt.

6. Vorrichtung nach Anspruch 5, wobei das Rückwandelement (34) die Rohrleitung (26) in einem im wesentlichen schraubenförmigen Bogen zu einer proximalen Stelle führt, die mit dem Magenentleerungsrohr (23) längsseitig des Luftwegrohrs (11) in Verbindung steht.

7. Vorrichtung nach einem der Ansprüche 1 bis 6, wobei die Rückkissenplatte tangential an der Rohrleitung befestigt (39) ist.

8. Vorrichtung nach Anspruch 7, wobei die Befestigung (39) des Rückkissens an der Rohrleitung über im wesentlichen die distale Hälfte der longitudinalen Erstreckung des aufweitbaren Rings verläuft

9. Vorrichtung nach einem der Ansprüche 1 bis 8, wobei die bogenförmigen Rippen (42) axialen Abstand voneinander aufweisen.

10. Vorrichtung nach Anspruch 9, wobei die Rippen (42) radial von der Rohrleitung nach außen ragen.

## Revendications

1. Un dispositif d'intubation avec masque laryngé comprenant:
un anneau gonflable généralement elliptique (18) en matériau élastomère moulé légèrement flexible et relativement mince, ayant une périphérie extérieure adaptée pour venir en prise de manière étanche avec l'entrée du larynx et comprenant dans sa périphérie intérieure un panneau perforé (33) séparant ses côtés pharyngé et laryngé, l'anneau s'étendant longitudinalement entre les extrémités proximale et distale et ayant un raccord d'orifice de gonflage (28) au niveau de son extrémité proximale, l'anneau comprenant en outre au niveau de son extrémité distale un conduit tubulaire ouvert (26) à des fins de mise en prise fonctionnelle et de communication avec l'entrée de l'oesophage, le conduit tubulaire s'étendant de son extrémité distalement ouverte de manière proximale sur le côté pharyngé du panneau;
une plaque de support en forme de dôme (34) en matériau relativement rigide ayant un côté concave se terminant en une base généralement elliptique dans un plan géométrique et fermé de manière étanche autour de l'ouverture dans le panneau (33) au niveau de la périphérie intérieure de l'anneau elliptique et ayant un raccord de sonde d'intubation proximal (36) formant un angle aigu avec le panneau, la plaque de support fournissant une stabilité à la périphérie intérieure de l'anneau elliptique et une stabilité directionnelle au conduit tubulaire;
une sonde d'intubation (11) connectée au raccord (36);
un tube de décharge gastrique (23) raccordé au conduit tubulaire (26); et
un coussinet arrière gonflable (25) comprenant un panneau en matériau légèrement élastique fixé de manière périphérique au côté pharyngé de l'anneau (18) et s'étendant sur le conduit tubulaire (26); **caractérisé en ce que**:
l'aspect postérieur du conduit tubulaire est relié sur sa longueur au panneau de coussinet arrière;
l'aspect antérieur du conduit tubulaire est relié (39) sur sa longueur à la plaque de support; et
les aspects latéraux du conduit tubulaire sont renforcés avec des nervures circonférentiellement incurvées (42).

2. Un dispositif selon la revendication 1, dans lequel la sonde d'intubation (11) et le tube de décharge gastrique (23) sont fixés ensemble côte à côte.

3. Un dispositif selon la revendication 1 ou la revendication 2, dans lequel le conduit tubulaire (26) s'étend de manière proximale jusqu'à 50 % de l'étendue longitudinale de l'anneau gonflable.

4. Un dispositif selon la revendication 1 ou la revendication 2, dans lequel le conduit tubulaire (26) s'étend de manière proximale sur plus de 50 % de l'étendue longitudinale de l'anneau gonflable.

5. Un dispositif selon l'une quelconque des revendications 1 à 4, dans lequel la plaque de support (34) guide le conduit tubulaire (26) dans une direction proximale rectiligne sur sensiblement la moitié distale de l'étendue longitudinale du masque.

6. Un dispositif selon la revendication 5, dans lequel l'élément formant plaque de support (34) guide le conduit tubulaire (26) sur un arc généralement hélicoïdal jusqu'à un emplacement proximal se raccordant au tube de décharge gastrique (23) à côté de la sonde d'intubation (11).

7. Un dispositif selon l'une quelconque des revendications 1 à 6, dans lequel le panneau de coussin arrière est fixé de manière tangentielle (39) au conduit tubulaire.

8. Un dispositif selon la revendication 7, dans lequel la fixation (39) du coussinet arrière au conduit tubulaire s'étend sur sensiblement la moitié distale de l'étendue longitudinale de l'anneau gonflable.

9. Un dispositif selon l'une quelconque des revendications 1 à 8, dans lequel les nervures incurvées (42) sont espacées axialement.

10. Un dispositif selon la revendication 9, dans lequel les nervures (42) font radialement saillie à l'extérieur du conduit tubulaire.
